Europäisches Patentamt
European Patent Office
Office européen des brevets

(11) Publication number: 0 182 135 A2

# EUROPEAN PATENT APPLICATION

(21) Application number: 85113419.7

(22) Date of filing: 23.10.85

(51) Int. Cl.⁴: C 07 C 97/26, C 07 C 103/44, C 07 C 157/02, A 61 K 31/22, A 61 K 31/16, A 61 K 31/17

(30) Priority: 19.11.84 US 673068

(43) Date of publication of application: 28.05.86 Bulletin 86/22

(84) Designated Contracting States: AT BE CH DE FR GB IT LI NL SE

(71) Applicant: AMERICAN CYANAMID COMPANY, 1937 West Main Street P.O. Box 60, Stamford Connecticut 06904 (US)

(72) Inventor: Murdock, Keith Chadwick, 15 Birch Street, Pearl River New York 10965 (US)

(74) Representative: Wächtershäuser, Günter, Dr., Tal 29, D-8000 München 2 (DE)

(54) **Novel bis-(substituted amino) anthraquinones.**

(57) Novel 1,4-bis-(substituted-amino) anthraquinones useful as sequestering agents and for inducing regression and/or palliation of cancer diseases in mammals.

EP 0 182 135 A2

29,786

Title: NOVEL BIS(SUBSTITUTED AMINO)ANTHRAQUINONES

This invention is concerned with novel 1,4-bis(substituted amino)anthraquinones which may be represented by the following general formula:

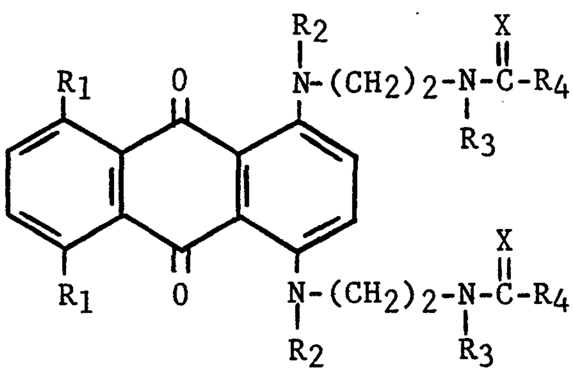

wherein $R_1$ is selected from the group consisting of hydroxy and $-O-\overset{\overset{O}{\|}}{C}-CF_3$; $R_2$ is selected from the group consisting of hydrogen and $-\overset{\overset{O}{\|}}{C}-CF_3$; $R_3$ is selected from the group consisting of hydrogen, $-CH_2CH_2-OH$, $-CH_2CH_2-O-\overset{\overset{O}{\|}}{C}-CH_3$ and $-CH_2CH_2-O-\overset{\overset{O}{\|}}{C}-CF_3$; $R_4$ is selected from the group consisting of hydrogen, trifluoromethyl, ethoxy, $-\overset{\overset{O}{\|}}{C}-CH_3$, $-\overset{\overset{O}{\|}}{C}-OCH_3$ and $-NHCH_2CH_2CH_2-N(CH_3)_2$; X is selected from the group consisting of oxygen and sulfur; and the pharmacologically acceptable acid-addition salts thereof.

The novel compounds of the present invention are obtainable as blue to blue-black crystalline solids having characteristic melting points and absorption spectra. The organic bases of this invention form non-toxic acid-addition salts when admixed with one or two equivalents of an acid in a neutral solvent. Such acids as sulfuric, phosphoric, hydrochloric, hydrobromic, sulfamic, citric, lactic, malic, succinic, tartaric, acetic, benzoic, gluconic, ascorbic and the like are preferred. For purposes of this invention the free bases are equivalent to their non-toxic acid-addition salts.

The novel compounds of the present invention may be prepared in accordance with the following reaction scheme:

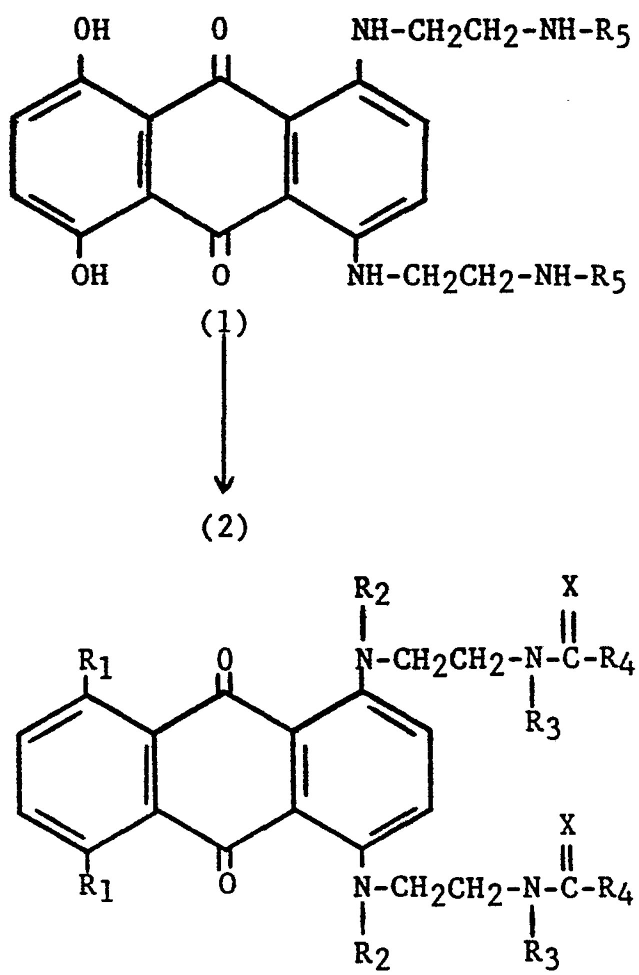

(1)

(2)

(2)

In accordance with the above reaction scheme, a 1,4-dihydroxyanthraquinone (1) where $R_5$ is hydrogen or B--hydroxyethyl is reacted with a variety of reagents as described below to produce the products (2) where $R_1$, $R_2$, $R_3$, $R_4$ and X are as described hereinabove.

Reaction of (1) where $R_5$ is $-CH_2CH_2OH$ with acetic anhydride in glacial acetic acid for 2-6 days gives (2) where $R_1$ is hydroxy, $R_2$ is hydrogen, $R_3$ is $-CH_2CH_2-O-\overset{O}{\overset{\|}{C}}-CH_3$, $R_4$ is methyl and X is oxygen.

Reaction of (1) where $R_5$ is $-CH_2CH_2OH$ with trifluoroacetic anhydride for 18-24 hours gives (2) where $R_1$ is $-O-\overset{O}{\overset{\|}{C}}-CF_3$, $R_2$ is $-\overset{O}{\overset{\|}{C}}-CF_3$, $R_3$ is $-CH_2CH_2-O-\overset{O}{\overset{\|}{C}}-CF$, $R_4$ is trifluoromethyl and X is oxygen.

Reaction of (1) where $R_5$ is hydrogen with di-methyl oxylate in N,N-dimethylformamide for 18-36 hours gives (2) where $R_1$ is hydroxy, $R_2$ is hydrogen, $R_3$ is hydrogen, $R_4$ is $-\overset{O}{\overset{\|}{C}}-OCH_3$ and X is oxygen.

Reaction of (1) where $R_5$ is hydrogen with ethyl chloroformate in pyridine at $0^oC$ for 1-4 hours gives (2) where $R_1$ is hydroxy, $R_2$ is hydrogen, $R_3$ is hydrogen, $R_4$ is ethoxy and X is oxygen.

Reaction of (1) where $R_5$ is hydrogen with ethyl chloroformate in pyridine at 0°C for 1-4 hours gives (2) where $R_1$, is hydroxy, $R_2$ is hydrogen, $R_3$ is hydrogen, $R_4$ is ethoxy and X is oxygen.

Reaction of (1) where $R_5$ is $-CH_2CH_2OH$ with 3--(dimethylamino)propylisothiocyanate in N,N-dimethylformamide for 4-8 hours at 30-40°C gives (2) where $R_1$ is hydroxy, $R_2$ is hydrogen, $R_3$ is hydroxyethyl, $R_4$ is $-NH-CH_2CH_2CH_2-N(CH_3)_2$ and X is sulfur. Treatment of an ethanol solution of this compound with ethanolic hydrogen chloride produces the dihydrochloride salt.

Reaction of (1) where $R_5$ is hydrogen with ethyl pyruvate in N, N-dimethylformamide for 2-4 hours gives (2) where $R_1$ is hydroxy, $R_2$ is hydrogen, $R_3$ is hydrogen, $R_4$ is $-C(=O)-CH_3$ and X is oxygen.

Reaction of (1) where $R_5$ is hydrogen with acetic-formic anhydride in formic acid for 1-2 hours gives (2) where $R_1$ is hydroxy, $R_2$, $R_3$ and $R_4$ are hydrogen and X is oxygen.

The novel compounds described herein are useful as chelating, complexing or sequestering agents. The complexes formed with polyvalent metal ions are particularly stable and usually soluble in various organic solvents. These properties render them useful for a variety of purposes wherein metal ion contamination presents a problem; e.g., as stabilizers in variuos organic systems such as saturated and unsaturated lubricating oils and hydrocarbons, fatty acids and waxes, wherein transition metal ion contamination accelerates oxidative deterioration and color formation. They are further useful in analyses of polyvalent metal ions which may be complexed or extracted by these materials and as metal carriers. Other uses common to sequestering agents are also apparent for these compounds.

The novel compounds of the present invention also possess the property of inducing regression and/or palliation of cancer diseases in mammals as established by the following tests.

Lymphocytic Leukemia P388 Test

The animals used were DBA/2 mice all of one sex, weighing a minimum of 17 g and all within a 3 g weight range. There were 5 or 6 animals per test group. The tumor transplant was by intraperitoneal injection of 0.1 ml of dilute ascitic fluid containing $10^6$ cells of lymphocytic leukemia P388. The test compounds were administered intraperitoneally on days 1, 5 and 9 (relative to tumor inoculation) at various doses. The animals were weighed and survivors were recorded on a regular basis for 30 days. The median survival time and the ratio of survival time for treated (T)/control (C) animals were calculated. The positive control compound was 1,4-dihydroxy-5,8-bis--[[2-(2-hydroxyethylamino)ethyl]amino]anthraquinone, dihydrochloride (U.S. Patent 4,197,249) given at the indicated doses by injection. The results of this test with representative compounds of the present invention appear in Table I.

TABLE I

Lymphocytic Leukemia P388 Test

| Compound | Dose (mg/kg) | Median Survival (Days) | T/C x 100 (%) |
|---|---|---|---|
| N,N'-[5,8-Dihydroxy-1,4-anthraquinonylenebis(imino-ethylene)][N-2-hydroxyethyl-acetamide-2,2'-diacetate] | 200<br>50<br>12<br>3<br>0.8 | 17<br>16<br>14<br>10<br>11 | 179<br>168<br>147<br>105<br>116 |
| Control | - | 9.5 | - |
| Positive Control | 3.2<br>1.6<br>0.8<br>0.4<br>0.2 | >30<br>>30<br>18<br>19.5<br>14.5 | >316<br>>316<br>189<br>205<br>153 |
| N,N'-(5,8-Dihydroxy-1,4-anthraquinonylene)bis[2,2,2-trifluoro-N-[2-(2,2,2-tri-fluoro-N-2-hydroxyethylacet-amido)ethyl]acetamide]-tetrakis(trifluoroacetate) (tetraester) | 6.4<br>1.6<br>0.4<br>0.1<br>0.025 | 26.5<br>19.5<br>15<br>16<br>13.5 | 230<br>170<br>130<br>139<br>117 |
| Control | - | 11.5 | - |
| Positive Control | 3.2<br>1.6<br>0.8<br>0.4<br>0.2 | >30<br>>30<br>18<br>19.5<br>14.5 | >261<br>>261<br>156<br>169<br>126 |

TABLE I (continued)

| Compound | Dose (mg/kg) | Median Survival (Days) | T/C x 100 (%) |
|---|---|---|---|
| 2,2'-[(9,10-Dihydro-5,8-dihydroxy-9,10-dioxo-1,4-anthracenediyl)bis(imino-2,1-ethanediylimino)]bis-[2-oxoacetic acid]dimethyl ester | 200<br>50<br>12<br>3 | 23<br>20.5<br>19.5<br>16 | 209<br>186<br>177<br>145 |
| Control | - | 11 | - |
| Positive Control | 1.6<br>0.4<br>0.1 | >30<br>>30<br>14.5 | >273<br>>273<br>132 |
| [(9,10-Dihydro-5,8-dihydroxy-9,10-dioxo-1,4-anthracenediyl)bis(imino-2,1-ethanediyl)]bis[carbamic acid]diethyl ester | 50<br>12<br>3<br>0.8<br>0.2 | 21.5<br>21<br>17<br>14<br>11 | 215<br>210<br>170<br>140<br>110 |
| Control | - | 10 | - |
| Positive Control | 1.6<br>0.4<br>0.1<br>0.025 | >30<br>27<br>25.5<br>17 | >300<br>270<br>255<br>170 |

TABLE I (continued)

| Compound | Dose (mg/kg) | Median Survival (Days) | T/C x 100 (%) |
|---|---|---|---|
| N,N"-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea | 200<br>50<br>12<br>3 | 23<br>19.5<br>13.5<br>13 | 219<br>186<br>129<br>124 |
| Control | - | 10.5 | - |
| Positive Control | 1.6<br>0.4<br>0.1<br>0.025 | >30<br>26.5<br>21.5<br>18 | >286<br>252<br>205<br>171 |
| N,N'-[(9,10-Dihydro-5,8-dihydroxy-9,10-dioxo-1,4-anthracenediyl)bis(imino-2,1-ethanediyl)]bis[2-oxo-propanamide] | 200<br>50 | 16.5<br>13.5 | 150<br>123 |
| Control | - | 11 | - |
| Positive Control | 1.6<br>0.4<br>0.1 | >30<br>19<br>16.5 | >273<br>173<br>150 |

TABLE I (continued)

| Compound | Dose (mg/kg) | Median Survival (Days) | T/C x 100 (%) |
|---|---|---|---|
| N,N"-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea, dihydrochloride | 50<br>12<br>3 | 22.5<br>19.5<br>15 | 205<br>177<br>136 |
| Control | - | 11 | - |
| Positive Control | 1.6<br>0.4<br>0.1<br>0.025 | 19.5<br>>30<br>25.5<br>16 | 177<br>>273<br>232<br>145 |

Melanotic Melanoma B16

The animals used were C57BC/6 mice, all of the same sex, weighing a minimum of 17 g and all within a 3 g weight range. There were normally 10 animals per test group. A 1.0 g portion of melanotic melanoma B16 tumor was homogenized in 10 ml of cold balanced salt solution and a 0.5 ml aliquot of the homogenate was implanted intraperitoneally into each of the test mice. The test compounds were administered intraperitoneally on days one through nine (relative to tumor inoculation) at various doses. The animals were weighed and survivors recorded on a regular basis for 60 days. The median survival time and the ratio of survival time for treated (T)/control (C) animals were calculated. The positive control compound was 1,4-dihydroxy-5,8-bis[[2-(2-hydroxyethylamino)-ethyl]amino]anthraquinone, dihydrochloride (U.S. Patent 4,197,249) given at the indicated doses by injection. The results of this test with representative compounds of the present invention appear in Table II.

TABLE II

Melanotic Melanoma B16 Test

| Compound | Dose (mg/kg) | Median Survival (Days) | T/C x 100 (%) |
|---|---|---|---|
| N,N'-[5,8-Dihydroxy-1,4-anthraquinonylenebis(iminoethylene)]bis[N-2-hydroxyethylacetamide-2,2'-diacetate] | 50<br>12<br>3<br>0.8 | 21.5<br>20.5<br>21.5<br>21.5 | 106<br>101<br>106<br>106 |
| Control | - | 20.2 | - |
| Positive Control | 1.6<br>0.8<br>0.4<br>0.2 | >60<br>35<br>36<br>32 | >297<br>173<br>178<br>158 |
| N,N'-(5,8-Dihydroxy-1,4-anthraquinonylene)bis[2,2,2-trifluoro-N-[2-(2,2,2-trifluoro-N-2-hydroxyethylacetamido)ethyl]acetamide]-tetrakis(trifluoroacetate)·(tetraester) | 12<br>3<br>0.8<br>0.2<br>0.05 | 42<br>>52<br>34<br>27<br>27 | 205<br>>254<br>166<br>132<br>132 |
| Control | - | 20.5 | - |
| Positive Control | 1.6<br>0.4<br>0.1<br>0.025 | >60<br>>60<br>36<br>28 | >293<br>>293<br>176<br>137 |

TABLE II (continued)

| Compound | Dose (mg/kg) | Median Survival (Days) | T/C x 100 (%) |
|---|---|---|---|
| 2,2'-[(9,10-Dihydro-5,8-dihydroxy-9,10-dioxo-1,4-anthracenediyl)bis(imino-2,1-ethanediylimino)]bis-[2-oxoacetic acid]dimethyl ester | 50<br>12<br>3<br>0.8 | >60<br>31.5<br>26.5<br>23.5 | >293<br>154<br>129<br>155 |
| Control | - | 20.5 | - |
| Positive Control | 1.6<br>0.4<br>0.1<br>0.025 | >60<br>>60<br>39<br>33 | >293<br>>293<br>190<br>161 |
| [(9,10-Dihydro-5,8-dihydroxy-9,10-dioxo-1,4-anthracenediyl)bis(imino-2,1-ethanediyl)]bis[carbamic acid]diethyl ester | 100<br>25<br>6<br>1.5 | 38.5<br>>60<br>39.5<br>29.5 | 204<br>>317<br>209<br>156 |
| Control | - | 18.9 | - |
| Positive Control | 1.6<br>0.4<br>0.1<br>0.025 | 38<br>>60<br>31.5<br>26 | 200<br>>317<br>166<br>137 |

TABLE II (continued)

| Compound | Dose (mg/kg) | Median Survival (Days) | T/C x 100 (%) |
|---|---|---|---|
| N,N"-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea | 100<br>25 | 42<br>27.5 | 222<br>146 |
| Control | - | 18.9 | - |
| Positive Control | 1.6<br>0.4<br>0.1<br>0.025 | 38<br>>60<br>31.5<br>26 | 200<br>>317<br>166<br>137 |
| N,N'-[(9,10-Dihydro-5,8-dihydroxy-9,10-dioxo-1,4-anthracenediyl)bis(imino-2,1-ethanediyl)]bis[2-oxo-propanamide] | 100<br>50<br>25 | 38<br>41<br>43.5 | 158<br>171<br>182 |
| Control | - | 24.0 | - |
| Positive Control | 1.6<br>0.4<br>0.1 | >60<br>>60<br>32 | >250<br>>250<br>133 |

TABLE II (continued)

| Compound | Dose (mg/kg) | Median Survival (Days) | T/C x 100 (%) |
|---|---|---|---|
| N,N"-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea, dihydrochloride | 12<br>3<br>0.8 | >60<br>33.5<br>24.5 | >279<br>156<br>114 |
| Control | - | 21.5 | - |
| Positive Control | 1.6<br>0.4<br>0.1<br>0.025 | 37<br>58<br>42<br>28.5 | 172<br>270<br>195<br>133 |

This aspect of the invention includes the novel compositions of matter and the method of inducing the regression and/or palliation of leukemia and related cancers in mammals when administered in amounts ranging from about one mg to about 1.2 g. per square meter of body surface area per day. The interrelationship of dosages for animals of various sizes and species and humans (based on $mg/m^2$ of surface area) is described by Freireich, E.J., et al., Quantitative Comparison of Toxicity of Anticancer Agents in Mouse, Rat, Hamster, Dog, Monkey and Man. Cancer Chemother. Rep., 50, No. 4, 219-244, May 1966. A preferred dosage regimen for optimum results would be from about 3 $mg/m^2$/day to about 200 $mg/m^2$/day, and such dosage units are employed that a total of from about 5 mg to about 360 mg of the active compound for a subject of about 70 kg of body weight are administered in a 24 hour period. This dosage regimen may be adjusted to provide the optimum therapeutic response. For example, several divided doses

may be administered daily or the dose may be proportionally reduced as indicated by the exigencies of the therapeutic situation. The active compound may be administered by the intravenous, intramuscular, or subcutaneous routes.

The active compounds may be administered parenterally or intraperitoneally. Solutions or dispersions of the active compound can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of micro--organisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases it will be preferable to include

isotonic agents, for example sugars of sodium chloride. Prolonged absorption of the injectable cmpositions can be brought about by the use in the compositions of agents delaying absorption, for example aluminum monostearate and gelatin. Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatable with the active ingredient, its use in the therapeutic compositions is contemplated. Supplementary active ingredients can also be incorporated into the compositions.

It is especially advantageous to formulate parenteral compositions in dosage unit form for each of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from about 2 mg to about 2 g, with from about 5 to about 360 mg being preferred. Expressed in proportions, the active compound is generally present in from about 2 to about 100 mg/ml of carrier. In the case of compositions containing supplementary active ingredients, the dosages are determined by reference to the usual dose and manner of administration of the said ingredients.

Regression and palliation of cancers are attained, for example, using intraperitoneal administration. A single intravenous dosage or repeated daily dosages can be administered. Daily dosages up to about 5 or 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia or the like, in the absence of excessive deleterious side effects of a cytotoxic nature to the hosts harboring the cancer. As used herein, cancer disease means blood malignancies such as leukemia, as well as other solid and non-solid malignancies such as the

melanocarcinomas, lung carcinomas, and mammary tumors. By regression and palliation is meant arresting or retarding the growth of the tumor or other manifestation of the disease compared to the course of the disease in the absence of treatment.

This invention will be described in greater detail in conjunction with the following specific examples.

EXAMPLE 1

N,N'-[5,8-Dihydroxyl,4-anthraquinonylene-bis(iminoethylene)]bis[N-2-hydroxyethylacetamide-2,2'diacetate]

A mixture of 30.0 g of 1,4-dihydroxy-5,8-bis--[[2-(2-hydroxyethylamino)ethyl]amino]anthraquinone dihydrochloride (U.S. Patent 4,197,249) and 300 ml of methanol was chilled in an ice bath in a Dewar flask. The mixture was saturated with ammonia gas and stirred at 0°C for one hour with the continuous slow addition of ammonia gas. The solid was collected and washed by slurrying with five 150 ml portions of ammonia saturated methanol, giving 22.9 g of 1,4-dihydroxy-5,8-bis[[2-(2-hydroxyethylamino)ethyl]amino]anthraquinone free base as blue-black microrods, mp 175-178°C.

To a solution of 3.0 g of the above free base in 30 ml of glacial acetic acid, obtained by warming at 95°C, then cooling, was added 30 ml of acetic anhydride. The solution was allowed to stand 4 days then poured into 350 ml of a mixture of ice and water, agitated vigorously and then allowed to stand at 5°C for 48 hours. The reaction mixture was extracted with three 100 ml portions of chloroform and each extract washed with three 100 ml portions of water. The chloroform extracts were combined, dried and evaporated in vacuo. The residue was dissolved in 40 ml of hot benzene and allowed to stand for 16 hours at room temperature. Seed crystals were produced by crystallizing a portion of the heavy immiscible oil in ether. These crystals were used to seed the main reaction

mixture in benzene. After standing 24 hours the solid was collected, washed with benzene, then ether and dried, giving 2.88 g of the desired product as a blue-black solid, mp 140-142°C.

EXAMPLE 2

N,N'-(5,8-Dihydroxy-1,4-anthraquinonylene)bis[2,2,2-trifluoro-N-[2,(2,2,2-trifluoro-N-2-hydroxyethyl-acetamido)ethyl]acetamide]tetrakis(trifluoroacetate) (tetraester)

A suspension of 1.37 g of 1,4-dihydroxy-5,8-bis-[[2-(hydroxyethylamino)ethyl]amino]anthraquinone free base in 10 ml of trifluoroacetic anhydride was stirred for 30 minutes, then the reaction vessel was stoppered, allowed to stand 21 hours and then evaporated to dryness *in vacuo* at <30°C. The residue was reevaporated three times with 20 ml portions of acetone and the final residue dried *in vacuo* at 40°C for 20 hours, giving 3.23 g of the desired product as a red-orange solid, mp 70°C.

EXAMPLE 3

2,2[(9-10-Dihydro-5,8-dihydroxy-9,10-dioxo-1,4-anthracenediyl)bis(imino-2,1-ethanediylimino)]-bis[2-oxoacetic acid]dimethyl ester

A 26.0 g amount of 1,4-bis[(2-aminoethyl)amino]--5,8-dihydroxyanthraquinone dihydrochloride [U.S. patent application, Serial No. 87,354, filed October 23, 1979 (ACCO Case No. 28,046)] was converted to the free base form by treating in aqueous solution with ammonium hydroxide.

A suspension of 2.5 g of the above free base in 35 ml of dry N,N-dimethylformamide containing 5.90 g of dimethyl oxalate was stirred for 24 hours. The resulting solid was collected and washed as follows, saving each washing separately: (A) 20 ml of N,N-dimethylformamide; (B) 15 ml of chloroform:methanol (3:1); (C,D,E) three times with 50 ml portions of chloroform:methanol (3:1). Examination of the washes by TLC on silica gel with tetrahydrofuran:water:acetic acid (4:2:1) showed that

B and C contained the desired product. These were evaporated, the solids combined and washed with acetone, giving 1.0 g of the desired product as a blue-black solid, mp 190-193°C.

EXAMPLE 4

[(9,10-Dihydro-5,8-dihydroxy-9,10-dioxo-1,4 anthracenediyl)bis(imino-2,1-ethanediyl)]bis-[carbamic acid]diethyl ester

To a stirred suspension of 2.5 g of 1,4-bis-[(2--aminoethyl)amino]-5,8-dihydroxyanthraquinone free base in 25 ml of pyridine at 0°C was added dropwise 1.43 ml of ethyl chloroformate. This mixture was stirred 2 hours at room temperature, then poured into a stirred mixture of ice and water. The resulting solid was collected, washed with water and dried, giving 2.75 g of the desired product as a dark blue solid, mp 124-129°C.

EXAMPLE 5

N,N"-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea

A mixture of 4.5 g of 1,4-dihydroxy-5,8-bis[[2--(2-hydroxyethylamino)ethyl]amino]anthraquinone free base and 3.0 g of 3-(dimethylamino)propylisothiocyanate in 50 ml of N,N-dimethylformamide was stirred for 6 hours at 35°C. The mixture was filtered and the filtrate evaporated to a syrup. The syrup was slurried with ether, the ether was decanted and the residue reslurried with ether, cooled and allowed to stand 16 hours. The supernatant was decanted and the procedure of reslurrying in ether, cooling and standing repeated. The solid was collected, giving 6.5 g of the desired product as a dark blue solid.

EXAMPLE 6

N,N'-[9,10-Dihydro-5,8-dihydroxy-9,10-dioxo-1,4-anthracenediyl)bis(imino-2,1-ethanediyl)]bis-[2-oxopropanamide]

A 2.5 g amount of 1,4-bis[(2-aminoethyl)amino]--5,8-dihydroxyanthraquinone free base was added to 5.81 g of ethyl pyruvate in 35 ml of N,N-dimethylformamide. The mixture was stirred for 2.5 hours and the solid was collected and washed with ether, giving 2.28 g of the desired product as a blue-black solid, mp >250°C.

EXAMPLE 7

N,N''-[(9,10-Dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea, dihydrochloride

A 4.0 g portion of the product of Example 5 was stirred with 110 ml of ethanol and then filtered. The filtrate was treated with 6 ml of 8N ethanolic hydrogen chloride and cooled. The resulting solid was collected, washed with ethanol and dried, giving 3.8 g of the desired product as a dark blue solid.

EXAMPLE 8

N,N'-[(9,10-Dihydro-5,8-dihydroxy-9,10-dixo-1,4,-anthracenediyl)bis(imino-2,1-ethanediyl)]bis-formamide

To a stirred suspension of 3.56 g of 1,4-bis[(2--aminoethyl)amino]-5,8- dihydroxyanthraquinone free base in 50 ml of 90% formic acid was added 7.0 ml of acetic-formic anhydride [J. Muramatsu, et al., Bul. Soc. Chem. Japan, 38, 244 (1945)]. This solution was stirred for one hour, then poured into 350 ml of water with stirring and made basic by the addition of ammonium hydroxide. The resulting solid was collected, air dried, then dried *in vacuo* at 75°C for 16 hours, giving 4.04 g of the desired product as a blue-black solid.

## CLAIMS

We claim:

1. A compound of the formula:

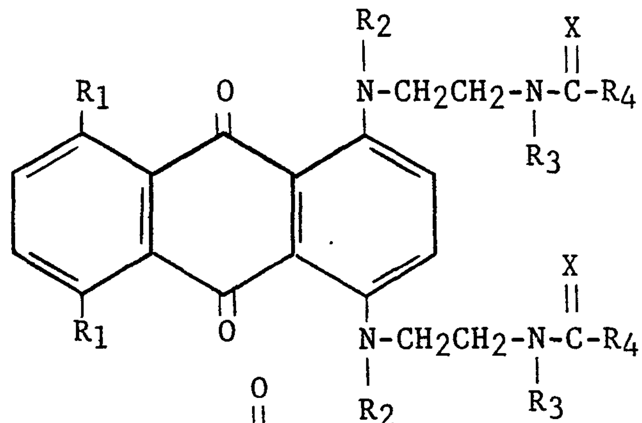

wherein $R_1$ is hydroxy or $-O-\overset{O}{\overset{||}{C}}-CF_3$; $R_2$ is hydrogen or $-\overset{O}{\overset{||}{C}}-CF_3$; $R_3$ is hydrogen, $-CH_2CH_2-OH$, $-CH_2CH_2-O-\overset{O}{\overset{||}{C}}-CH_3$ or $-CH_2CH_2-O-\overset{O}{\overset{||}{C}}-CF_3$; $R_4$ is hydrogen, methyl, trifluoromethyl, ethyoxy $-\overset{O}{\overset{||}{C}}-CH_3$, $-\overset{O}{\overset{||}{C}}-OCH_3$ or $-NHCH_2CH_2CH_2-N(CH_3)_2$; X is oxygen or sulfur; and the pharmacologically acceptable acid-addition salts thereof.

2. The compound according to Claim 1; N,N'--[5,8-dihydroxy-1,4-anthraquinonylenebis(iminoethylene)]-bis[N-2-hydroxyethylacetamide-2,2"-diacetate].

3. The compound according to Claim 1; N,N'--(5,8-dihdyroxy-1,4-anthraquinonylene)bis[2,2,2-trifluoro--N-[2-(2,2,2-trifluoro-N-2-hydroxethylacetamido)ethyl]-acetamide]tetrakis(trifluoroacetate)(tetraester).

4. The compound according to Claim 1; 2,2'--[(9,10-dihdyro-5,8-dihydroxy-9,10-dioxo-1,4--anthracenediyl)bis(imino-2,1-ethanedihylimino)]bis[2--oxoacetic acid]dimethyl ester.

5. The compound according to Claim 1; [(9,10--dihydro-5,8-dihydroxy-9,10-dioxo-1,4-anthracenediyl)bis-(imino-2,1-ethanediyl)]bis[carbamic acid]diethyl ester.

6. The compound according to Claim 1; N,N"-[(9,10-dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea.

7. The compound according to Claim 1; N,N'-[(9,10-dihydro-5,8-dihydroxy-9,10-dioxo-1,4-anthracenediyl)bis(imino-2,1-ethanediyl)]bis[2-oxopropanamide].

8. The compound according to Claim 1; N,N"-[(9,10-dihydro-5,8-dihydroxy-9,10-dioxo-1,4-anthracenediyl)bis(imino-2,1-ethanediyl)]bis[N'-[3-(dimethylamino)propyl]-N-(2-hydroxyethyl)]thiourea, dihydrochloride.

9. The compound according to Claim 1; N,N'-[(9,10-dihydro-5,8-dihydroxy-9,10-dioxo-1,4-anthracenediyl)bis(imino-2,1-ethanediyl)]bis-formamide.

10. A composition of matter is dosage unit form comprising from about 3 $mg/m^2$ to about 200 $mg/m^2$ of body surface area of a compound selected from those of the formula:

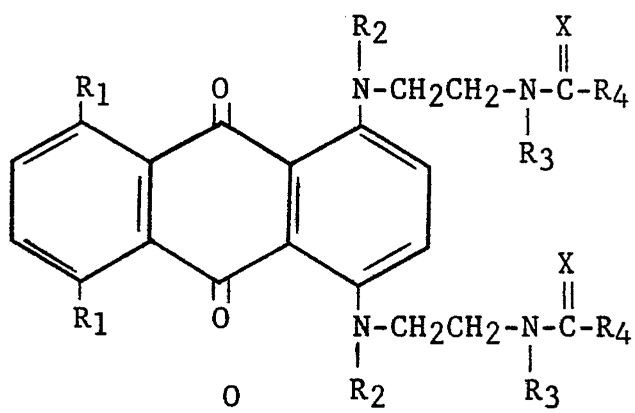

wherein $R_1$ is hydroxy or $-O-\overset{\overset{O}{\|}}{C}-CF_3$; $R_2$ is hydrogen or $-\overset{\overset{O}{\|}}{C}-CF_3$; $R_3$ is hydrogen $-CH_2CH_2-OH$, $-CH_2CH_2-O-\overset{\overset{O}{\|}}{C}-CH_3$ or $-CH_2CH_2-O-\overset{\overset{O}{\|}}{C}-CF_3$; $R_4$ is hydrogen, methyl, trifluoromethyl, ethyoxy $-\overset{\overset{O}{\|}}{C}-CH_3$, $-\overset{\overset{O}{\|}}{C}-OCH_3$ or $-NHCH_2CH_2CH_2-N(CH_3)_2$; X is oxygen or sulfur; and the pharmacologically acceptable acid-addition salts thereof.